# NEW EUROPEAN PATENT SPECIFICATION

(11) **EP 1 230 375 B2**
(45) Date of publication and mention of the opposition decision: **26.10.2016**
(45) Mention of the grant of the patent: 06.07.2005
(21) Application number: 00976188.3
(22) Date of filing: 17.11.2000
(51) Int. Cl.: C12N 15/63, C12N 15/11, A61K 31/713, A01K 67/027, C12N 5/10

(54) **INHIBITING GENE EXPRESSION WITH DSRNA**
HEMMUNG DER EXPRESSION VON GENEN MITTELS DSRNA
INHIBITION D'EXPRESSION GENIQUE A L'AIDE D'ARN BICATENAIRE

(30) Priority: 19.11.1999 GB 9927444
(43) Date of publication of application: 14.08.2002
(60) Divisional application: 04022291.1
(73) Proprietor: Cancer Research Technology Limited, London WC2A 3PX (GB)
(72) Inventor: Zernicka-Goetz, Magdelena Cambridge House, London NW1 4JL (GB); Wianny, Florence Cambridge House, London NW1 4JL (GB); Evans, Martin John Cambridge House, London NW1 4JL (GB); Glover, David Moore University of Cambridge,, Cambridge, CB2 3DY (GB)
(74) Representative: Vossius & Partner Patentanwälte Rechtsanwälte mbB
(86) International application number: PCT/GB2000/004404
(87) International publication number: WO 2001/036646

(56) References cited:
- EP-A- 1 197 567
- WO-A-00/01846
- WO-A-00/44914
- WO-A-00/63364
- WO-A-97/07668
- WO-A-98/05770
- WO-A-99/32619
- FIRE A: "RNA-triggered gene silencing" TRENDS IN GENETICS, vol. 15, no. 9, 1 September 1999 (1999-09-01), pages 358-363, XP004176656 ISSN: 0168-9525 cited in the application
- BEVILACQUA A ET AL: "ANTISENSE RNA INHIBITS ENDOGENOUS GENE EXPRESSION IN MOUSE PREIMPLANTATION EMBRYOS LACK OF DOUBLE-STRANDED RNA MELTING ACTIVITY" PROCEEDINGS OF THE NATIONAL ACADEMY OF SCIENCES OF THE UNITED STATES, vol. 85, no. 3, 1988, pages 831-835, XP002164293 ISSN: 0027-8424 cited in the application
- ZERNICKA-GOETZ M ET AL: "Following cell fate in the living mouse embryo" DEVELOPMENT, vol. 124, no. 6, 1 March 1997 (1997-03-01), pages 1133-1137, XP002086618 ISSN: 0950-1991 cited in the application
- WIANNY F ET AL: "Specific interference with gene function by double-stranded RNA in early mouse development" NATURE CELL BIOLOGY, vol. 2, no. 2, February 2000 (2000-02), pages 70-75, XP002138445 ISSN: 1465-7392

## Description

The present invention relates to inhibiting gene expression. In particular, it relates to inhibiting gene expression in mammals using double stranded RNA (dsRNA).

The benefits of being able to inhibit the expression of a specific gene or group of genes in mammals are obvious. Many diseases (such as cancer, endocrine disorders, immune disorders and so on) arise from the abnormal expression of a particular gene or group of genes within a mammal - the inhibition of the gene or group can therefore be used to treat these conditions. Similarity, disease can result through expression of a mutant form of protein, m which case it would be advantageous to eliminate the expression of the mutant allele. In addition, such gene specific inhibition may be used to treat viral diseases which are caused by for example retroviruses, such as HIV, in which viral genes are integrated into the genome of their host and expressed.

In addition, the elimination or inhibition of expression of a specific gene can be used to study and manipulate early developmental events in the non-human embryo. The most valuable information would be obtained if the function of the gene of interest could be disturbed in specific cells of the embryo and at defined times. In such a situation, in the mouse model, the classical techniques of gene "knockout" cannot be used, because they eliminate gene function universally throughout the embryo. Furthermore, if a gene is repeatedly used in space and time to direct developmental processes, elimination of its role by conventional gene "knockout" may deny an understanding of everything but the first event. Even when the interest is to study the very first time in development at which a gene functions, the contribution of maternal transcripts and their translation products can mask the effects of the gene knockout. Existing "knockout" technology is also extremely laborious. It necessitates first making a disrupted gene segment that is suitably marked to enable the selection of homologous recombination events in cultured embryonic stem cells. Such cells must then be incorporated into blastocysts and the resulting chimaeric animals used to establish pure breeding lines before homozygous mutants can be obtained.

It is known that expression of genes can be specifically inhibited by double stranded RNA in certain organisms. Double stranded RNA interference (RNAi) of gene expression was first shown in *Caenorhabditis elegans* (Fire et al. Nature 391, 806-811 (1998); WO99/32619), has recently been shown to be effective in lower eukaryotes including *Drosophila melanogaster* (Kennerdell. & Carthew, Cell 95, 1017-1026 (1998)), *Trypanosoma brucei* (Ngo, et al. Proc Natl Acad Sci USA 95, 14687-14692 (1998)), planarians (Sanchez Alvarado & Newmark, Proc Natl Acad Sci U S A 96, 5049-5054 (1999)) and plants (Waterhouse, et al. Proc Natl Acad Sci U S A 95, 13959-13964 (1998)). The application of this approach has also been demonstrated in Zebrafish embryos, but with limited success (Wargelius, et al. Biochem Biophys Res Commun 263, 156-161 (1999)).

To date, there has been no report that RNAi can be used in mammals and moreover there is a belief in the art that RNAi will not function in mammals. In this respect, concern has been expressed that the protocols used for invertebrate and plant systems are unlikely to be effective in mammals (reviewed by Fire (Fire Trends Genet15,358-363

(1999)). This is because accumulation of dsRNA in mammalian cells can result in a general block to protein synthesis. The accumulation of very small amounts of double stranded RNA (dsRNA) in mammalian cells following viral infection results in the interferon response (Marcus, Interferon 5, 115-180 (1983)) which leads to an overall block to translation and the onset of apoptosis (Lee & Esteban Virology 199, 491-496 (1994)). Part of the interferon response is the activation of a dsRNA responsive protein kinase (PKR) (Clemens, Int JBiochem Cell Biol 29, 945-949 (1997)). This enzyme phosphorylates and inactivates translation factor EIF2α in response to dsRNA. The consequence is a global suppression of translation, which in turn triggers apoptosis. Wagner & Sun. (Nature 391, 806-811 (1998)) suggest that RNAi will not work in mammals because it has no effect when used as a control in experiments into anti-sense RNA.

Anti-sense RNA has been attempted as a means of reducing gene expression in the embryos of a number of species. Whereas it has had considerable success in Drosophila, it has been disappointing in Zebrafish, Xenopus and mouse embryos. In Xenopus, there were some limitations in using the antisense approach. This is thought to be due to a prominent RNA melting activity (Bass, & Weintraub, Cell 48, 607-613 (1987); Rebaghati & Melton, Cell 48, 599-605 (1987)), exerted by the dsRNA specific adenosine deaminase (dsRAD), and suggests that RNAi is not likely to be successful.

In the mouse embryo, anti-sense RNA has had inconsistent and limited success in reducing gene expression, particularly between the two-four cell stages (Bevilacqua, et al. Proc Natl Acad Sci USA 85, 831-835 (1988)). These authors were concerned that the partial inhibition of β*-glucuronidase* in their experiments might also reflect a melting activity acting upon sense/anti-sense duplexes, and so they examined the stability of β*-glucuronidase* dsRNA microinjected into mouse blastomeres. They reported no effects on RNA stability, but this was only followed over a period of 5 hours. Thus, there is no suggestion in this paper that dsRNA can persist in mammalian cells long enough to interfere with gene expression. In addition, they reported no effects upon the expression of β*-glucuronidase* following the injection of dsRNA. Thus, this paper does not suggest that dsRNA can inhibit gene expression in mammalian cells.

WO99/32619 suggests that dsRNA can be used to inhibit gene expression in mammals. However, the only experimental evidence in this document shows that RNAi works in *C. elegans*; there is nothing to show that it could work in mammals. Indeed, later publications by the inventors listed for WO99/32619 (Fire, Trends Genet 15, 358-363 (1999), (Montgomery & Fire, Trends Genet 14, 255-258 (1998)) state that RNAi could only be made to work in mammals if the PKR response could be neutralised or some way avoided, although no suggestions are provided in WO99/32619 for how this might be achieved. These later publications indicate that the inventors of WO99/32619 themselves believe that RNAi has not yet been (and cannot be) made to work in mammals.

Thus, there is a perception in the art that RNAi cannot be made to work in mammals. Contrary to this perception, the inventors have now shown that it is possible to interfere with specific gene expression in the mouse oocyte and zygote following microinjection of the appropriate dsRNA. They have shown experimentally that RNAi can phenocopy the effects of disrupting the maternal expression of the c-*mos* gene in the oocyte to overcome the arrest of meiosis at metaphase II, or the zygotic expression of *E-cadherin* to prevent development of the blastocyst as observed in the corresponding knockout mice. The inventors have shown that the injection of a dsRNA is specific to the corresponding gene; it does not cause a general translational arrest, because embryos continue to develop and no signs of cell death can be observed. Thus, they have shown that RNAi can be effective in mammalian cells. The invention is defined by the claims. Accordingly, provided is the use of an RNA in the manufacture of a medicament for inhibiting the expression of a target gene in a mammalian cell, wherein the target gene causes or is likely to cause disease and wherein the mammalian cell is an embryonic stem cell of a non-human pre-implantation embryo, the RNA comprising a double stranded structure having a nucleotide sequence, which nucleotide sequence is 100% identical to at least a part of said target gene in said mammalian cell which double stranded structure is at least 25 bases long, and which nucleotide sequence is derived from an endogenous template, and wherein the RNA comprises two separate complementary RNA strands.

Described is a RNA comprising a double stranded structure having a nucleotide sequence which is at least 80% identical to at least a part of a target gene in a mammalian cell or is capable of hybridising with a portion of the target gene transcript under the following conditions: 400 mM NaCl, 40 mM PIPES pH 6.4, 1 mM EDTA, 50°C or 70°C hybridisation for 12-16 hours, followed by washing, and which is derived from an endogenous template, or an expression vector encoding such an RNA, for use as a medicament.

Described is an *in vitro* method for inhibiting the expression of a target gene in a mammalian cell, the method comprising:
introducing into the cell an RNA comprising a double stranded structure having a nucleotide sequence which is at least 80% identical to at least a part of the target gene or is capable of hybridising with a portion of the target gene transcript under the following conditions: 400 mM NaCl, 40 mM PIPES pH 6.4, 1 mM EDTA, 50°C or 70°C hybridization for 12-16 hours, followed by washing, and which is derived from an endogenous template; and verifying inhibition of expression of the target gene,
wherein the mammalian cell is a somatic cell, a non-human oocyte or a cell of a non-human pre-implantation embryo.

RNA, also referred to as dsRNA, which is useful in accordance with the invention is derived from an "endogenous template". Such a template may be all or part of a nucleotide sequence endogenous to the mammal; it may be a DNA gene sequence or a cDNA produced from an mRNA isolated from the mammal, for example by reverse transcriptase. When the template is all or a part of a DNA gene sequence, it is preferred if it is from one or more or all exons of the gene. Additionally, all or part of a viral gene may form an endogenous template, if it is expressed in the mammal in such a way that the interferon response is not induced, e.g. expression from a pro-virus integrated into the host cell chromosome. Thus, the dsRNA of the present invention is distinguished from viral dsRNA and synthetic polyrlC, both of which have been observed to induce PKR which leads to apoptosis in mammalian cells.

Whilst the dsRNA is derived from an endogenous template, there is no limitation on the manner in which it is synthesised. Thus, it may be synthesised *in vitro* or *in vivo,* using manual and/or automated procedures. *In vitro* synthesis may be chemical or enzymatic, for example using cloned RNA polymerase (e.g., T3, T7, SP6) for transcription of the endogenous DNA (or cDNA) template, or a mixture of both.

*In vivo,* the dsRNA may be synthesised using recombinant techniques well known in the art (see e.g., Sambrook, et al., MOLECULAR CLONING; A LABORATORY MANUAL, SECOND EDITION (1989); DNA CLONING, VOLUMES I AND II (D. N Glover ed. 1985); OLIGONUCLEOTIDE SYNTHESIS (M. J. Gait ed, 1984); NUCLEIC ACID HYBRIDISATION (B. D. Hames & S. J. Higgins eds. 1984); TRANSCRIPTION AND TRANSLATION (B. D. Hames & S. J. Higgins eds. 1984), ANIMAL CELL CULTURE (R. I. Freshney ed. 1986); IMMOBILISED CELLS AND ENZYMES (IRL Press, 1986); B. Perbal, A PRACTICAL GUIDE TO MOLECULAR CLONING (1984); the series, METHODS IN ENZYMOLOGY (Academic Press, Inc.); GENE TRANSFER VECTORS FOR MAMMALIAN CELLS (J. H. Miller and M. P. Calos eds. 1987, Cold Spring Harbor Laboratory), Methods in Enzymology Vol. 154 and Vol. 155 (Wu and Grossman, and Wu, eds., respectively), Mayer and Walker, eds. (1987), IMMUNOCHEMICAL METHODS IN CELL AND MOLECULAR BIOLOGY (Academic Press, London), Scopes, (1987), PROTEIN PURIFICATION: PRINCIPLES AND PRACTICE, Second Edition (Springer-Verlag, N.Y.),and HANDBOOK OF EXPERIMENTAL IMMUNOLOGY, VOLUMES I-IV (D. M. Weir and C C. Blackwell eds 1986).

Thus, bacterial cells can be transformed with an expression vector which comprises the DNA template from which the dsRNA is to be derived. Alternatively, the cells of the mammal in which inhibition of gene expression is required may be transformed with an expression vector or by other means. Bidirectional transcription of one or more copies of the template may be by endogenous RNA polymerase of the transformed cell or by a cloned RNA polymerase (e.g., T3, T7, SP6) coded for by the expression vector or a different expression vector. The use and production of an expression construct are known in the art (see WO98/32016; US Pat Nos. 5,593,874, 5,698,425, 5712,135, 5,789,214, and 5,804,693). Inhibition of gene expression may be targeted by specific transcription in an organ, tissue, or cell type; an environmental condition (e.g. infection, stress, temperature, chemical); and/or engineering transcription at a developmental stage or age, especially when the dsRNA is synthesised *in vivo* in the mammal. dsRNA may also be delivered to specific tissues or cell types using known gene delivery systems. Known eukaryotic vectors include pWLNEO, pSV2CAT, pOG44, pXT1 and pSG available from Stratagene; and pSVK3, pBPV, pMSG and pSVL available from Pharmacia. These vectors are listed solely by way of illustration of the many commercially available and well known vectors that are available to those of skill in the art.

If synthesised outside the mammalian cell, the RNA may be purified prior to introduction into the cell. Purification may be by extraction with a solvent (such as phenol/chloroform) or resin, precipitation (for example in ethanol), electrophoresis, chromatography, or a combination thereof. However, purification may result in loss of dsRNA and may therefore be minimal or not carried out at all. The RNA may be dried for storage or dissolved in an aqueous solution, which may contain buffers or salts to promote annealing, and/or stabilisation of the RNA strands.

dsRNA useful in the present invention includes dsRNA which contains one or more modified bases, and dsRNA with a backbone modified for stability or for other reasons. For example, the phosphodiester linkages of natural RNA may be modified to include at least one of a nitrogen or sulphur heteroatom. Moreover, dsRNA comprising unusual bases, such as inosine, or modified bases, such as tritylated bases, to name just two examples, can be used in the invention. It will be appreciated that a great variety of modifications have been made to RNA that serve many useful purposes known to those of skill in the art. The term dsRNA as it is employed herein embraces such chemically, enzymatically or metabolically modified forms of dsRNA, provided that it is derived from an endogenous template.

The double stranded structure may be formed by a single self-complementary RNA strand or two separate complementary RNA strands. RNA duplex formation may be initiated either inside or outside the mammalian cell.

The dsRNA comprises a double stranded structure, the sequence of which is at least 100% identical to at least a part of the target gene. "Identity", as known in the art, is the relationship between two or more polynucleotide (or polypeptide) sequences, as determined by comparing the sequences. In the art, identity also means the degree of sequence relatedness between polynucleotide sequences, as determined by the match between strings of such sequences. Identity can be readily calculated *(*Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds.,MStockton Press, New York, 1991). While there exist a number of methods to measure identity between two polynucleotide sequences, the term is well known to skilled artisans *(*Sequence Analysis in Molecular Biology, von Heinje, G., Academic Press, 1987; Sequence Analysis Primer, Gribskov, M. and Devereux, J., eds., M Stockton Press, New York, 1991; and Carillo, H., and Lipman, D., SIAM J. Applied Math., 48: 1073 (1988).

Methods commonly employed to determine identity between sequences include, but are not limited to those disclosed in Carillo, H., and Lipman, D., SIAM J. Applied Math., 48:1073 (1988). Preferred methods to determine identity are designed to give the largest match between the sequences tested. Methods to determine identity are codified in computer programs. Computer program methods to determine identity between two sequences include, but are not limited to, GCG program package (Devereux, J., et al., Nucleic Acids Research 12(1): 387 (1984)), BLASTP, BLASTN, and FASTA (Atschul, S.F. et al., J. Molec. Biol. 215: 403 (1990)). Another software package well known in the art for carrying out this procedure is the CLUSTAL program. It compares the sequences of two polynucleotides and finds the optimal alignment by inserting spaces in either sequence as appropriate. The identity for an optimal alignment can also be calculated using a software package such as BLASTx. This program aligns the largest stretch of similar sequence and assigns a value to the fit. For any one pattern comparison several regions of similarity may be found, each having a different score. One skilled in the art will appreciate that two polynucleotides of different lengths may be compared over the entire length of the longer fragment. Alternatively small regions may be compared. Normally sequences of the same length are compared for a useful comparison to be made.

There is 100% sequence identity between the inhibitory RNA and the part of the target gene. However, dsRNA having 80% or greater than 90% or 95% sequence identity are described, and thus sequence variations that might be expected due to genetic mutation, strain polymorphism, or evolutionary divergence can be tolerated.

The duplex region of the RNA may have a nucleotide sequence that is capable of hybridising with a portion of the target gene transcript (e.g., 400 mM NaCl, 40 mM PIPES pH 6.4, 1 mM EDTA, 50°C or 70°C hybridisation for 12-16 hours; followed by washing).

Whilst the optimum length of the dsRNA may vary according to the target gene and experimental conditions, the duplex region of the RNA is at least 25 bases long.

As used herein "target gene" generally means a polynucleotide comprising a region that encodes a polypeptide, or a polynucleotide region that regulates replication, transcription or translation or other processes important to expression of the polypeptide, or a polynucleotide comprising both a region that encodes a polypeptide and a region operably linked thereto that regulates expression. Target genes may be cellular genes present in the genome or viral and pro-viral genes that do not elicit the interferon response, such as retroviral genes. The target gene may be a protein-coding gene or a non-protein coding gene, such as a gene which codes for ribosmal RNAs, splicosomal RNA, tRNAs, etc.

The dsRNA may be identical to the whole of the target gene, i.e. the coding portion of the gene. However, the dsRNA can be identical to a part of the target gene. The size of this part depends on the particular target gene and can be determined by those skilled in the art by varying the size of the dsRNA and observing whether expression of the gene has been inhibited.

The dsRNA can be used to inhibit a target gene which causes or is likely to cause disease, i.e. it can be used for the treatment or prevention of disease. In the prevention of disease, the target gene may be one which is required for initiation or maintenance of the disease, or which has been identified as being associated with a higher risk of contracting the disease.

In the treatment of disease, the dsRNA can be brought into contact with the cells or tissue exhibiting the disease. For example, dsRNA substantially identical to all or part of a mutated gene associated with cancer, or one expressed at high levels in tumour cells, e.g. aurora kinase, may be brought into contact with or introduced into a cancerous cell or tumour gene. Examples of cancers include solid tumours and leukaemias, including: apudoma, choristoma, branchioma, malignant carcinoid syndrome, carcinoid heart disease, carcinoma (e.g.,Walker, basal cell, basosquamous, Brown-Pearce, ductal, Ehrlich tumour, *in situ,* Krebs 2, Merkel cell, mucinous, non-small cell lung, oat cell, papillary, scirrhous, bronchiolar, bronchogenic, squamous cell, and transitional cell), histiocytic disorders, leukaemia (e.g., B cell, mixed cell, null cell, T cell, T-cell chronic, HTLV-IIassociated, lymphocytic acute, lymphocytic chronic, mast cell, and myeloid), histiocytosis malignant, Hodgkin disease, immunoproliferative small, non-Hodgkin lymphoma, plasmacytoma, reticuloendotheliosis, melanoma, chondroblastoma, chondroma, chondrosarcoma, fibroma, fibrosarcoma, giant cell tumours, histiocytoma, lipoma, liposarcoma, mesothelioma, myxoma, myxosarcoma, osteoma, osteosarcoma, Ewing sarcoma, synovioma, adenofibroma, adenolymphoma, carcinosarcoma, chordoma, cranio-pharyngioma, dysgerminoma, hamartoma, mesenchymoma, mesonephroma, myosarcoma, ameloblastoma, cementoma, odontoma, teratoma, thymoma, trophoblastic tumour, adeno-carcinoma, adenoma, cholangioma, cholesteatoma, cylindroma, cystadenocarcinoma, cystadenoma, granulosa cell tumour, gynandroblastoma, hepatoma, hidradenoma, islet cell tumour, Leydig cell tumour, papilloma, Sertoli cell tumour, theca cell tumour, leiomyoma, leiomyosarcoma, myoblastoma, mymoma, myosarcoma, rhabdomyoma, rhabdomyosarcoma, ependymoma, ganglioneuroma, glioma, medulloblastoma, meningioma, neurilemmoma, neuroblastoma, neuroepithelioma, neurofibroma, neuroma, paraganglioma, paraganglioma nonchromaffin, angiokeratoma, angiolymphoid hyperplasia with eosinophilia, angioma sclerosing, angiomatosis, glomangioma, hemangioendothelioma, hemangioma, hemangiopericytoma, hemangiosarcoma, lymphangioma, lymphangiomyoma, lymphangiosarcoma, pinealoma, carcinosarcoma, chondrosarcoma, cystosarcoma, phyllodes, fibrosarcoma, hemangiosarcoma, leimyosarcoma, leukosarcoma, liposarcoma, lymphangiosarcoma, myosarcoma, myxosarcoma, ovarian carcinoma, rhabdomyosarcoma, sarcoma (e.g., Ewing, experimental, Kaposi, and mast cell), neoplasms (e.g., bone, breast, digestive system, colorectal, liver, pancreatic, pituitary, testicular, orbital, head and neck, central nervous system, acoustic, pelvic respiratory tract, and urogenital), neurofibromatosis, and cervical dysplasia, and other conditions in which cells have become immortalized or transformed. The invention could be used in combination with other treatments, such as chemotherapy, cryotherapy, hyperthermia, radiation therapy, and the like.

Described is also the treatment and prophylaxis of other diseases, especially those associated with expression or overexpression of a particular gene or genes. For example, expression of genes associated with the immune response could be inhibited to treat/prevent autoimmune diseases such as rheumatoid arthritis, graft-versus-host disease, etc. In such treatment, the dsRNA may be used in conjunction with immunosuppressive drugs. The most commonly used immunosuppressive drugs currently include corticosteroids and more potent inhibitors like, for instance, methotrexate, sulphasalazine, hydroxychloroquine, 6-MP/azathioprine and cyclosporine. All of these treatments have severe side-effects related to toxicity, however, and the need for drugs that would allow their elimination from, or reduction in use is urgent. Other immunosuppressive drugs include the gentler, but less powerful non-steroid treatments such as Aspirin and Ibuprofen, and a new class of reagents which are based on more specific immune modulator functions. This latter class includes interleukins, cytokines, recombinant adhesion molecules and monoclonal antibodies. The use of dsRNA to inhibit a gene associated with the immune response in an immunosuppressive treatment protocol could increase the efficiency of immunosuppression, and particularly, may enable the administered amounts of other drugs, which have toxic or other adverse effects to be decreased.

The following classes of possible target genes are examples of the genes which the present invention may be used to inhibit: developmental genes (e.g., adhesion molecules cyclin kinase inhibitors, Wnt family members, Pax family members, Winged helix family members, Hox family members, cytokines/lymphokines and their receptors, growth/differentiation factors and their receptors, neurotransmitters and their receptors); oncogenes (e.g., ABLI, BCL1, BCL2, BCL6, CBFA2, CBL, CSFIR, ERBA, ERBB, EBRB2, ETS1, ETS1, ETV6, FGR, FOS, FYN, HCR, HRAS, JUN, KRAS, LCK, LYN, MDM2, MLL, MYB, MYC, MYCL1, MYCN, NRAS, PIM1, PML, RET, SRC, TAL1, TCL3 and YES); tumour suppresser genes (e.g., APC, BRCA1, BRCA2, MADH4, MCC, NF1, NF2, RB1, TP53 and WT1); and enzymes (e.g., ACP desaturases and hydroxylases, ADP-glucose pyrophorylases, ATPases, alcohol dehydrogenases, amylases, amyloglucosidases, catalases, cellulases, cyclooxygenases, decarboxylases, dextrinases, DNA and RNA polymerases, galactosidases, glucanases, glucose oxidases, GTPases, helicases, hemicellulases, integrases, invertases, isomerases, kinases, lactases, lipases, lipoxygenases, lysozymes, pectinesterases, peroxidases, phosphatases, phospholipases, phosphorylases, polygalacturonases, proteinases and peptideases, pullanases, recombinases, reverse transcriptases, topoisomerases, and xylanases).

Described is that the dsRNA is not derived from β*-glucuronidase.* Also described is an *in vitro* method for inhibiting the expression of a target gene in a mammalian cell, the method comprising:
introducing into the cell an RNA comprising a double stranded structure having a nucleotide sequence which is at least 80% identical to at least a part of the target gene or is capable of hybridising with a portion of the target gene transcript under the following conditions: 400 mM NaCl, 40 mM PIPES pH 6.4, 1 mM EDTA, 50°C or 70°C hybridization for 12-16 hours, followed by washing, and which is derived from an endogenous template, wherein the dsRNA is not derived from β*-glucuronidase* wherein the mammalian cell is a somatic cell, a non-human oocyte or a cell of a non-human pre-implantation embryo.

Inhibition of the expression of a target gene can be verified by observing or detecting an absence or observable decrease in the level of protein encoded by a target gene (this may be detected by for example a specific antibody or other techniques known to the skilled person) and/or mRNA product from a target gene (this may be detected by for example hybridisation studies) and/or phenotype associated with expression of the gene. In the context of a medical treatment, verification of inhibition of the expression of a target gene may be by observing a change in the disease condition of a subject, such as a reduction in symptoms, remission, a change in the disease state and so on. Preferably, the inhibition is specific, i.e. the expression of the target gene is inhibited without manifest effects on the other genes of the cell.

The amount of dsRNA administered to a mammal for effective gene inhibition will vary between wide limits according to a variety of factors, including the route of administration, the age, size and condition of the mammal, the gene which is to be inhibited, the disease or disorder to be treated and so on. The present inventors have found that, when injecting 10 pl into a non-human oocyte or cell of the non-human early embryo, solutions having dsRNA at a concentration in the range of from 0.01 to 40 mg/ml, preferably 0.1 to 4 mg/ml and most preferable 0.1 to 2 mg/ml are effective. Thus, the dsRNA may be administered to provide 0.1 to 400 pg, preferably 1 to 40 pg and most preferably 1 to 20 pg in each cell.

The cell having the target gene may be somatic, totipotent or pluripotent, dividing or non-dividing, epithelium, immortalised or transformed, from the non-human germ line, or the like. The cell may be a stem cell or a differentiated cell. Cell types that are differentiated include adipocytes, fibroblasts, myocytes, cardiomyocytes, endothelium, neurons, glia, blood cells, megakaryoctyes, lymphocytes, macrophages, neutrophils, eosinophils, basophils, mast cells, leukocytes, granulocytes, keratinocytes, chondrocytes, osteoblasts, osteoclasts, hepatocytes, and cells of the endocrine or exocrine glands. The cell may be any individual cell of the non-human early embryo, and may be a non-human blastocyte. Alternatively, it may be a non-human oocyte. It is known that mammalian cells can respond to extracellular dsRNA and therefore may have a transport mechanism for dsRNA (Asher et al, Nature 223 715-717 (1969)). Thus dsRNA may be administered extracellularly into a cavity, interstitial space, into the circulation of a mammal, or introduced orally. Methods for oral introduction include direct mixing of the RNA with food of the mammal, as well as engineered approaches in which a species that is used as food is engineered to express the RNA, then fed to the mammal to be affected. For example, food bacteria, such as *Lactococcus lactis,* may be transformed to produce the dsRNA (see WO93/17117, WO97/14806). Vascular or extravascular circulation, the blood or lymph systems and the cerebrospinal fluid are sites where the RNA may be injected.

RNA may be introduced into the cell intracellularly. Physical methods of introducing nucleic acids may also be used in this respect. The dsRNA may be administered using the microinjection techniques described in Zernicka-Goetz,. et al. Development 124, 1133-1137 (1997) and Wianny, et al. Chromosoma 107, 430-439 (1998).

Other physical methods of introducing nucleic acids intracellularly include bombardment by particles covered by the RNA, for example gene gun technology in which the dsRNA is immobilised on gold particles and fired directly at the site of wounding. Thus, described is the use of an RNA comprising a double stranded structure having a nucleotide sequence, which is substantially identical to at least a part of a target gene in a mammalian cell and which is derived from an endogenous template, in a gene gun for inhibiting the expression of the target gene. Further, there is provided a composition suitable for gene gun therapy comprising: an RNA comprising a double stranded structure having a nucleotide sequence which is substantially identical to at least a part of a target gene in a mammalian cell and which is derived from an endogenous template; and gold particles. An alternative physical method includes electroporation of cell membranes in the presence of the RNA. dsRNA can be introduced into non-human embryonic cells by electoporation using conditions similar to those generally applied to cultured cells. Precise conditions for electroporation depend on the device used to produce the electro-shock and the dimensions of the chamber used to hold the embryos. This method permits RNAi on a large scale. Any known gene therapy technique can be used to administer the RNA. A viral construct packaged into a viral particle would accomplish both efficient introduction of an expression construct into the cell and transcription of RNA encoded by the expression construct. Other methods known in the art for introducing nucleic acids to cells may be used, such as lipid-mediated carrier transport, chemical-mediated transport, such as calcium phosphate, and the like. Thus, the RNA may be introduced along with components that perform one or more of the following activities: enhance RNA uptake by the cell, promote annealing of the duplex strands, stabilize the annealed strands, or otherwise increase inhibition of the target gene. A non-human transgenic mammal that expresses RNA from a recombinant construct may be produced by introducing the construct into a zygote, an embryonic stem cell, or another multipotent cell derived from the appropriate mammal.

Described is the use of an RNA, or expression vector encoding such an RNA, as defined in the first aspect, in the manufacture of a medicament for inhibiting the expression of a target gene in a mammalian cell, wherein the target gene causes or is likely to cause disease and wherein the mammalian cell is a somatic cell or a cell of a non-human pre-implantation embryo.

The medicament will usually be supplied as part of a sterile, pharmaceutical composition which will normally include a pharmaceutically acceptable carrier. Thus, described is a pharmaceutical formulation comprising an RNA as defined in the first aspect, or an expression vector encoding such an RNA, together with a pharmaceutically acceptable carrier.

This pharmaceutical composition may be in any suitable form (depending upon the desired method of administering it to a patient). It may be provided in unit dosage form, will generally be provided in a sealed container and may be provided as part of a kit.

Such a kit would normally (although not necessarily) include instructions for use. It may include a plurality of said unit dosage forms.

The pharmaceutical composition may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such compositions may be prepared by any method known in the art of pharmacy, for example by admixing the active ingredient with the camer(s) or excipient(s) under sterile conditions.

Pharmaceutical compositions adapted for oral administration may be presented as discrete units such as capsules or tablets; as powders or granules; as solutions, syrups or suspensions (in aqueous or non-aqueous liquids; or as edible foams or whips; or as emulsions). Suitable excipients for tablets or hard gelatine capsules include lactose, maize starch or derivatives thereof, stearic acid or salts thereof. Suitable excipients for use with soft gelatine capsules include for example vegetable oils, waxes, fats, semisolid, or liquid polyols etc.

For the preparation of solutions and syrups, excipients which may be used include for example water, polyols and sugars. For the preparation of suspensions oils (e.g. vegetable oils) may be used to provide oil-in-water or water-in-oil suspensions.

Pharmaceutical compositions adapted for topical administration may be formulated as ointments, creams, suspensions, lotions, powders, solutions, pastes, gels, sprays, aerosols or oils. For infections of the eye or other external tissues, for example mouth and skin, the compositions are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base. Pharmaceutical compositions adapted for topical administration to the eye include eye drops wherein the active ingredient is dissolved or suspended in a suitable carrier, especially an aqueous solvent. Pharmaceutical compositions adapted for topical administration in the mouth include lozenges, pastilles and mouth washes.

Pharmaceutical compositions adapted for rectal administration may be presented as suppositories or enemas.

Pharmaceutical compositions adapted for nasal administration wherein the carrier is a solid include a coarse powder having a particle size for example in the range 20 to 500 µm which is administered in the manner in which snuff is taken, i.e. by rapid inhalation through the nasal passage from a container of the powder held close up to the nose. Suitable compositions wherein the carrier is a liquid, for administration as a nasal spray or as nasal drops, include aqueous or oil solutions of the active ingredient.

Pharmaceutical compositions adapted for administration by inhalation include fine particle dusts or mists which may be generated by means of various types of metered dose pressurised aerosols, nebulizers or insufflators.

Pharmaceutical compositions adapted for vaginal administration may be presented as pessaries, tampons, creams, gels, pastes, foams or spray formulations.

Pharmaceutical compositions adapted for parenteral administration include aqueous and non-aqueous sterile injection solution which may contain anti-oxidants, buffers, bacteriostats and solutes which render the formulation substantially isotonic with the blood of the intended recipient; and aqueous and non-aqueous sterile suspensions which may include suspending agents and thickening agents. Excipients which may be used for injectable solutions include water, alcohols, polyols, glycerine and vegetable oils, for example. The compositions may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets.

The pharmaceutical compositions may contain preserving agents, solubilising agents, stabilising agents, wetting agents, emulsifiers, sweeteners, colourants, odourants, salts (substances of the present invention may themselves be provided in the form of a pharmaceutically acceptable salt), buffers, coating agents or antioxidants. They may also contain therapeutically active agents in addition to the substance of the present invention.

Dosages of the substance of the present invention can vary between wide limits, depending upon the disease or disorder to be treated, the age and condition of the individual to be treated, etc. and a physician will ultimately determine appropriate dosages to be used. This dosage may be repeated as often as appropriate. If side effects develop the amount and/or frequency of the dosage can be reduced, in accordance with normal clinical practice.

The described dsRNA may be used alone or as a component of a kit having at least one of the reagents necessary to carry out the *in vitro* or *in vivo* introduction of RNA to subjects. Preferred components are the dsRNA and a vehicle that promotes introduction of the dsRNA. Such a kit may also include instructions to allow a user of the kit to practice the invention.

Described is a kit for inhibiting expression of a target gene in a mammalian cell, the kit comprising:
RNA which comprises a double stranded structure having a nucleotide sequence which is at least 80% identical to at least a part of a target gene in the mammalian cell or is capable of hybridising with a portion of the target gene transcript under the following conditions: 400 mM NaCl, 40 mM PIPES pH 6.4, 1 mM EDTA, 50°C or 70°C hybridisation for 12-16 hours, followed by washing, and which is derived from an endogenous template or an expression vector encoding such an RNA; and
a vehicle that promotes introduction of the RNA or vector to the mammalian cell,
wherein the RNA is not derived from the gene encoding β-glucuronidase and wherein the mammalian cell is a somatic cell, a non-human oocyte or a cell of a non-human pre-implantation embryo.

Described is also manipulating gene expression in the non-human oocyte to treat infertility. Described is also regulating the processes of chromosome disjunction in non-human mammals. In humans, there is an increased incidence of chromosome non-disjunction in mothers over 35 years of age, leading to Downs syndrome offspring and spontaneous abortion. A number of cell cycle regulatory molecules are now known that promote several aspects of cycle progression that include cyclin dependent kinases, cyclins, polo kinase, aurora kinase, min A kinase, protein phosphatases, compounds of the anaphase promoting complex and its regulatory molecules, compounds of the proteosome, the SCF complex, compounds of the centrosome, components of the kinetochore, structural proteins of chromosomes, DNA replication enzymes, DNA recombination proteins and DNA repair proteins. The invention may be used in non-humans to modulate the expression of one or more of the above proteins to ensure correct segregation of chromosomes.

Described is also manipulating the cell cycle stages of recipient enucleated zygotes and donor cells that provide the nuclei for the cloning of non-human mammals (see WO97/07668). Experience with the cloning of sheep and mice shows a need to optimise the cell cycle stage of the recipient egg prior to its enucleation, and to take down nuclei from cells at a specific stage, frequently, but not necessarily, Gₒ cells. Application of the present invention to arrest one or more of the cell cycle molecules indicated above may be used to this end.

Described is also directing patterns of gene expression in pluripotent cells in order to produce specific differentiated cell types for use in transplantation to replace diseased or otherwise non-functional tissue. One example of pluripotent cells are the embryonic stem (ES) cells from non-human pre-implantation embryos. It is well known in the art that mouse ES cells can be reintroduced into the blastocyst whereupon they become incorporated into the developing embryo, develop and differentiate into all bodily cell types and structures. ES cells can also be induced to differentiate *in vitro* into a wide range of cell types following the removal of specific growth factors from the culture medium. It is expected that ES cell lines can be established from all mammals and indeed methods for establishing human ES cell lines have already been established. The differentiation of pluripotent cell types into specific cell types requires that certain pathways of gene expression are turned off and others are turned on. The present invention can be applied to eliminate key proteins within such regulatory pathways in order to direct ES and other embryonic cells to differentiate into specific cell types. Described is also interfering with the expression of developmental genes (such as those mentioned herein) to direct cell differentiation along preferred pathways. It is also known that certain cell types complete their differentiation upon exit from the cell division cycle. Described is also inhibiting cell cycle regulatory molecules, such as those listed above. These dsRNAs may be used directly or expressed from regulatable promoters to effect the final stages of cell differentiation.

Described is also an isolated mammalian cell containing an expression construct, the construct coding for an RNA which forms a double stranded structure having a nucleotide sequence which is at least 80% identical to at least a part of a target gene or is capable of hybridising with a portion of the target gene transcript under the following conditions: 400 mM NaCl, 40 mM PIPES pH 6.4, 1 mM EDTA, 50°C or 70°C hybridisation for 12-16 hours, followed by washing, and which is derived from an endogenous template, wherein the mammalian cell is a somatic cell, a non-human oocyte or a cell of a non-human pre-implantation embryo.

When used herein, "treatment/therapy" includes any regime that can benefit a human or non-human animal, and "comprising/having" covers anything consisting only of a specified feature/characteristic, as well as anything with that feature/characteristic, but which also has one or more additional features/characteristics.

### Examples

The present invention will now be described further in the following examples. Reference is made to the accompanying drawings:

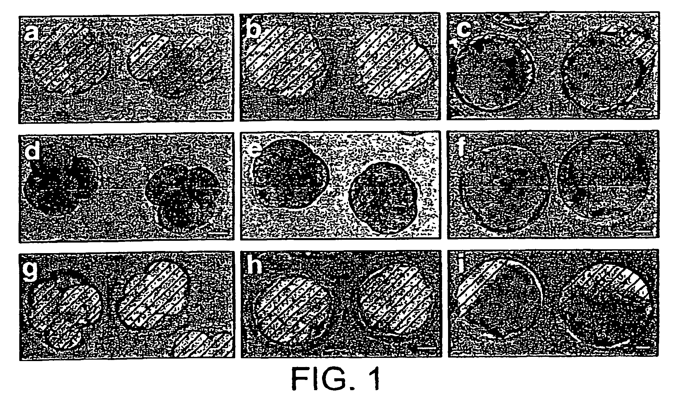

Figure 1: *MmGFP* dsRNA specifically abrogates MmGFP expression in MmGFP transgenic embryos
   **(a-c)** Representative embryos out of 131 embryos obtained from eleven different matings between F1 females and MmGFP transgenic males. MmGFP transgenic 4-6 cell stage embryos **(a),** morula **(b),** blastocysts **(c).** A similar pattern of GFP expression was obtained after injection of antisense *MmGFP* RNA. **(d-f)** Representative embryos out of 147 MmGFP transgenic embryos that had been injected with *MmGFP* dsRNA at the one cell stage. 4-6 cell stage embryos **(d),** morula **(e),** blastocyst **(f). (g-i)** Representative embryos out of 18 MmGFP transgenic embryos that had been injected with *c-mos* dsRNA at the one cell stage. 6 cell stage embryos **(g),** morula **(h),** blastocyst **(i).** Scale bars represent 20 µm. The shading indicates green fluorescence.
Figure 2: Interference with expression of injected synthetic *MmGFP* mRNA.
   (a), Wild type morulae injected with *MmGFP* mRNA alone; **(b),** together with *E-cadherin* dsRNA; and (c), together with *MmGFP* dsRNA, at the one cell stage. Scale bars represent 20 µm. The shading indicates green fluorescence.
Figure 3: Injection of *E-cadherin* dsRNA to the zygote reduces *E-cadherin* expression and perturbs the development of the injected embryos.
   (a), Immunofluorescent staining of *E-cadherin* in embryos injected at the one-cell stage with *MmGFP* dsRNA, and cultured for four days *in vitro* until the blastocyst stage. (b), Immunofluorescent staining of *E-cadherin* in embryos injected at the one-cell stage with *E-cadherin* dsRNA, and cultured for four days *in vitro.* Note the altered development of these embryos. Scale bars represent 20 µm. (c), Western blot analysis of *E-cadherin* expression in zygotes, uninjected morulae (collected at the one-cell stage and cultured *in vitro* for three days), morulae injected at the one-cell stage with 2 mg ml⁻¹ of *GFP* dsRNA and cultured *in vitro* for three days, morulae injected at the one-cell stage with 2 mg ml⁻¹ of *E-cadherin* dsRNA and cultured *in vitro* for three days. In each case, proteins were extracted from 15 embryos. This experiment has been repeated three times with the same result. The reduction of signal following *E-cadherin* dsRNA injection was approximately 6.5 fold. Scale bars represent 20 µm. The shading indicates chemiluminescence.

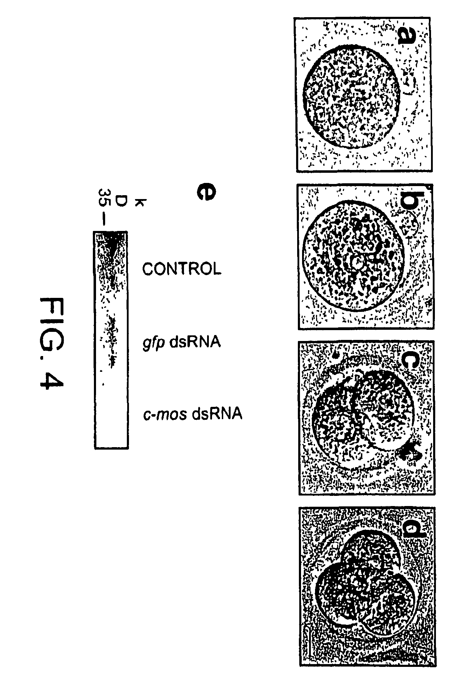

Figure 4: Injection of c-mos dsRNA in immature oocyte inhibits c-mos expression and causes parthenogenetic activation.
   **(a-d)** Examples of parthenogenetically activated eggs obtained after injection of *c-mos* dsRNA in germinal vesicle stage oocytes. **(a),** Control oocyte arrested in metaphase II; **(b),** one-cell embryo (white arrow points out the pronucleus); **(c),** two-cell embryo; **(d),** four cell embryo. Scale bars represent 20 µm. **(e),** Western blot analysis of c-mos expression in oocytes arrested in metaphase II, oocytes injected at the germinal vesicle stage with 2 mg ml⁻¹ of *MmGFP* dsRNA and cultured *in vitro* during 12 hours, oocytes injected at the germinal vesicle stage with 2 mg ml⁻¹ of c-mos dsRNA and cultured *in vitro* during 12 hours. In each case, proteins were extracted from 35 oocytes. This experiment has been repeated two times with the same result.

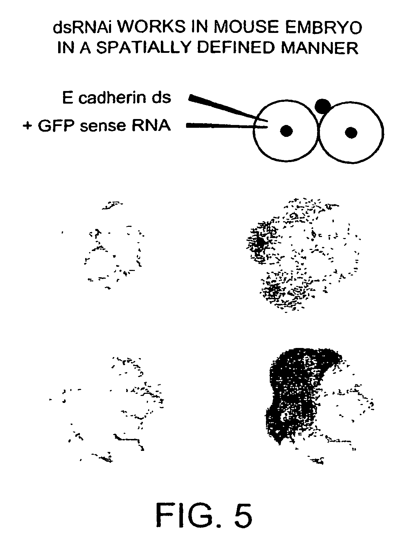

Figure 5: Inhibition of gene expression following injection of double stranded RNA is restricted to the clonal lineage derived from the injected cell.

ways. It is also known that certain cell types complete their differentiation upon exit from the cell division cycle. The invention may therefore also be used to inhibit cell cycle regulatory molecules, such as those listed above. These dsRNAs may be used directly or expressed from regulatable promoters to effect the final stages of cell differentiation.

The invention also provides an isolated mammalian cell containing an expression construct, the construct coding for an RNA which forms a double stranded structure having a nucleotide sequence which is at least 80% identical to at least a part of a target gene or is capable of hybridising with a portion of the target gene transcript under the following conditions: 400 mM NaCl, 40 mM PIPES pH 6.4, 1 mM EDTA, 50°C or 70°C hybridisation for 12-16 hours, followed by washing, and which is derived from an endogenous template, wherein the mammalian cell is a somatic cell, a non-human oocyte or a cell of a non-human pre-implantation embryo.

When used herein, "treatment/therapy" includes any regime that can benefit a human or non-human animal, and "comprising/having" covers anything consisting only of a specified feature/characteristic, as well as anything with that feature/characteristic, but which also has one or more additional features/characteristics.

Preferred features of each aspect of the invention are as for each of the other aspects *mutatis mutandis.*

### Examples

The present invention will now be described further in the following examples. Reference is made to the accompanying drawings:
Figure 1: *MmGFP* dsRNA specifically abrogates MmGFP expression in MmGFP transgenic embryos
   **(a-c)** Representative embryos out of 131 embryos obtained from eleven different matings between F1 females and MmGFP transgenic males. MmGFP transgenic 4-6 cell stage embryos **(a),** morula **(b),** blastocysts **(c).** A similar pattern of GFP expression was obtained after injection of antisense *MmGFP* RNA. **(d-f)** Representative embryos out of 147 MmGFP transgenic embryos that had been injected with *MmGFP* dsRNA at the one cell stage. 4-6 cell stage embryos (d), morula **(e),** blastocyst **(f). (g-i)** Representative embryos out of 18 MmGFP transgenic embryos that had been injected with *c-mos* dsRNA at the one cell stage. 6 cell stage embryos **(g),** morula **(h),** blastocyst **(i).** Scale bars represent 20 µm. The shading indicates green fluorescence.

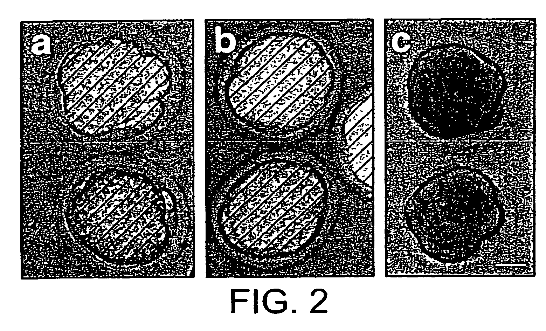

Figure 2: Interference with expression of injected synthetic *MmGFP* mRNA.
   **(a),** Wild type morulae injected with *MmGFP* mRNA alone; (b), together with *ECadherin* dsRNA; and **(c),** together with *MmGFP* dsRNA, at the one cell stage. Scale bars represent 20µm. The shading indicates green fluorescence.

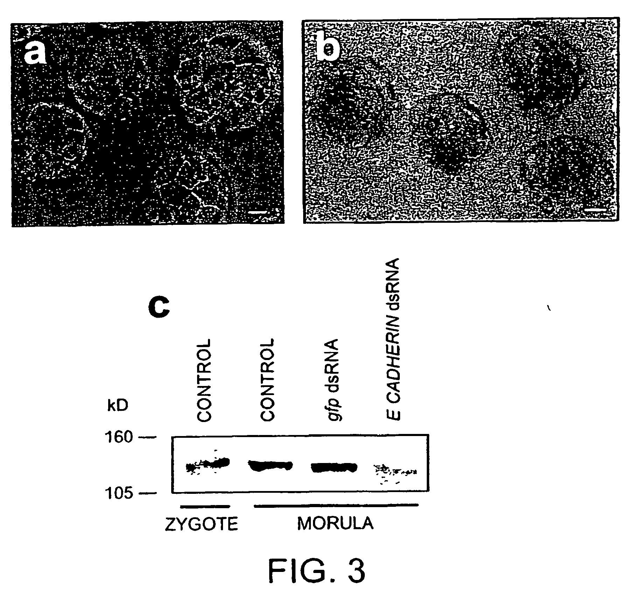

Figure 3: Injection of *E-cadherin* dsRNA to the zygote reduces E-cadherin expression and perturbs the development of the injected embryos.
   **(a),** Immunofluorescent staining of E-cadherin in embryos injected at the one-cell stage with *MmGFP dsRNA,* and cultured for four days *in vitro* until the blastocyst stage. **(b),** Immunofluorescent staining of E-cadherin in embryos injected at the one-cell stage with *E-cadherin* dsRNA, and cultured for four days *in vitro.* Note the altered development of these embryos. Scale bars represent 20µm. **(c),** Western blot analysis of E-cadherin expression in zygotes, uninjected morulae (collected at the one-cell stage and cultured *in vitro for* three days), morulae injected at the one-cell stage with 2 mg ml⁻¹ of GFP dsRNA and cultured *in vitro* for three days, morulae injected at the one-cell stage with 2 mg ml⁻¹ of *E-cadherin* dsRNA and cultured *in vitro* for three days. In each case, proteins were extracted from 15 embryos. This experiment has been repeated three times with the same result. The reduction of signal following *E-cadherin* dsRNA injection was approximately 6.5 fold. Scale bars represent 20µm. The shading indicates chemiluminescence.
Figure 4: Injection of *c-mos* dsRNA in immature oocyte inhibits c-mos expression and causes parthenogenetic activation.
   **(a-d)** Examples of parthenogenetically activated eggs obtained after injection of *c-mos* dsRNA in germinal vesicle stage oocytes. **(a),** Control oocyte arrested in metaphase II; **(b),** one-cell embryo (white arrow points out the pronucleus); **(c),** two-cell embryo; **(d),** four cell embryo. Scale bars represent 20µm. **(e),** Western blot analysis of c-mos expression in oocytes arrested in metaphase II, oocytes injected at the germinal vesicle stage with 2 mg ml⁻¹ of *MmGFP* dsRNA and cultured *in vitro* during 12 hours, oocytes injected at the germinal vesicle stage with 2 mg ml⁻¹ of *c-mos* dsRNA and cultured *in vitro* during 12 hours. In each case, proteins were extracted from 35 oocytes. This experiment has been repeated two times with the same result.
Figure 5: Inhibition of gene expression following injection of double stranded RNA is restricted to the clonal lineage derived from the injected cell.
Immunofluoresecent staining of E-cadherin in embryos injected in one cell at the two cell stage with *E-cadherin* dsRNA and synthetic mRNA for MmGFP. The left hand panels show single channel (red) fluorescence to reveal E-Cadherin. Note that the staining is markedly reduced in the progeny of the injected cell. These progeny cells are identified in the corresponding second (green) channels as cells expressing MmGFP.

### Methods

### Collection and culture of oocytes and embryos

Immature oocytes arrested at prophase I of meiosis were collected from ovaries of 4-6-week-old F₁ (CBAxC57BI) mice in FHM medium (Speciality media, Inc. Lavalette, N.J.) supplemented with Bovine Serum Albumin (BSA) (4 mg ml⁻¹). F1 female mice were superovulated by intraperitoneal injections of pregnant mare's serum gonadotrophin (PMSG, 5 i.u) and human chorionic gonadotrophin (hCG) 48-52 hours apart. Fertilised 1 cell embryos were obtained from mated females 20-24 hours after hCG.

### RNA synthesis and microinjections

The templates used for RNA synthesis were linearised plasmids. Full length *MmGFP* cDNA (714bp) was cloned into T7TS plasmid (Zernicka-Goetz,. etal. Development 124, 1133-1137 (1997)). A Kpnl/Hindlll fragment of c-*mos* cDNA (550bp) (Colledge etal, Nature 370, 665-68 (1994)) was cloned into Bluescript pSK. A cDNA corresponding to *exon4-exon8* of *E-cadherin* (580bp) (Larue et al, Proc Nat Acad Sci USA 92, 855-859 (1995)) was cloned into Bluescript pKS. RNAs were synthesised using the T3 or T7 polymerases, using the Megascripts kit (Ambion). DNA templates were removed with DNAse treatment. The RNA products were extracted with phenol/chloroform, and ethanol precipitated.

To anneal, equimolar quantities of sense and antisense RNA were mixed in the annealing buffer (10mM Tris pH7.4, EDTA 0.1 mM) to a final concentration of 2 µM each, heated for 10 min at 68°C, and incubated at 37°C for 3-4 hrs. To avoid the presence of contaminating single stranded RNA in the dsRNA samples, the preparations were treated with 2µg/ml of RNase T1 (Calbiochem) and 1 µg/ml RNase A (Sigma) for 30 min at 37°C. The dsRNAs were then treated with 140 µg/ml proteinase K (Sigma), phenol/chloroform extracted and ethanol precipitated. Formation of dsRNA was confirmed by migration on an agarose gel: for each dsRNA, the gel mobility was shifted compared to the ssRNAs. For comparison of antisense and double-stranded RNAs, equal masses of RNA were infected.

RNAs were diluted in water, to a final concentration of 2 to 4 mg ml⁻¹. The range of effective concentrations is best illustrated by the c-mos experiment (Table 2) due to the sensitivity of this biological phenotype. The mRNAs were microinjected into the cytoplasm of the oocytes or embryos, using a constant flow system (Transjector, Eppendorf) as described (Zernicka-Goetz in Cell lineage and fate determination (ed. Moody, S. A.) 521-527 (Academic Press, San Diego, CA, 1999)). Each oocyte or embryo was injected with approximately 10pl of dsRNA. Improved penetrance was achieved by using negative capacitance. After microinjection, oocytes and embryos were cultured in KSOM (Speciality media, Inc. Lavalette, N.J.) medium supplemented with 4 mg ml⁻¹ of BSA, at 37°C in a 5 % CO₂ atmosphere. MmGFP transgenic embryos were observed by confocal microscopy (Biorad 1024 scanning head on a Nikon Eclipse 800 microscope).

### Immunoblot and immunostaining analysts

For immunoblot analysis, samples were subjected to SDS-polyacrylamide gel electrophoresis and proteins were transferred to a hybond nitrocellulose membrane (Amersham). Membranes were preincubated in TBST buffer (20mM Tris-HCl, pH8.2, 150mM NaCl, 0.1 % Tween-20) containing 5 % (w/v) non-fat dried milk overnight, to block non-specific binding of antibodies. They were then incubated with the anti-E cadherin antibody (DECMA-1) or the antimos antibody (SantaCruz Biotechnology), during 1 hour, washed in TBST, and incubated with the peroxidase conjugated secondary antibody (SantaCruz Biotechnology) for 1 hour, and washed again in TBST The antibodies were diluted in TBST containing 5% (w/v) non fat dried milk. The secondary antibody was detected by enhanced chemiluminescence (Amersham). For whole mount immunofluorescence with E-cadherin antibody, embryos were fixed in 2 % paraformaldehyde for 20 min at room temperature, followed by permeabilization with 0.1% Triton X-100 for 10 min. After preincubation in 2 % BSA in PBS for 30 min, embryos were incubated with the anti-E cadherin antibody for 1 hour at 37°C, and with a Texas-Red conjugated goat anti-rat antibody (Jackson ImmunoResearch Laboratories, West Grove, Pa., USA), for 1 hour at 37°C. Embryos were observed using the Biorad 1024 laser scanning confocal microscope.

### Example 1 - dsRNA prevents gfp transgene expression

To determine whether dsRNA might be used to prevent gene expression in the mouse embryo, we developed an experimental test system using a transgenic strain of mice that expresses MmG FP under the control of the Elongation Factor 1 α (E1Fα) promoter (Zernicka-Goetz, M. in Cell lineage and fate determination (ed. Moody, S. A.) 521-527 (Academic Press, San Diego, CA, 1999)). This line offered the advantage that GFP expression can be easily visualised in living embryos and, because its function is non-essential, we could monitor any non-specific deleterious effects of dsRNA on embryonic development. In order to avoid the complication of perdurance of maternal gene products, we used heterozygous embryos in which the transgene was paternally derived. The onset of GFP expression in these embryos is seen by the appearance of green cells following the initiation of zygotic transcription at the two cell stage.

We were able to demonstrate that the injection of *MmGFP* dsRNA into the single cell zygote prevented the onset of the appearance of green fluorescence at the 2-4 cell stages (Fig. 1). After injection, embryos were cultured *in vitro* for 3-4 days to the blastocyst stage. While uninjected embryos expressed MmGFP in the expected manner (Fig. 1a-c), all embyros the injected with Mn dsRNA showed a dramatically decreased green fluorescence throughout this period (Fig.1d-f), with a minorproportion (6.8%) showing residual green fluorescence. The embryos showed normal pre- and postimplantation development, demonstrating that the injection of dsRNA is not toxic.

The interference with gene expression is specific because, when we injected an unrelated dsRNA corresponding to a segment of the *c-mos* transcript into MmGFP transgenic embryos, this did not result in a decrease in green fluorescence (Fig 1g-i). Similarly, injection of dsRNA corresponding to a segment of E-cadherin transcript into transgenic zygotes (59 embryos observed) did not result in a decrease in green fluorescence, and did not shut down protein synthesis via dsRNA kinase, although the genotype of such embryos was abnormal (data not shown, see below). We also found thattransgenic zygotes injected with antisense MnRNA retain the green fluorescence at all pre-implanatation stages (37 embryos observed - data not shown).

We also attempted to determine whether expression of MmGFP from capped full length MmGFPmRNA could be eliminated by the co-injection of MmGFPdsRNA.

We found that green fluorescence was greatly diminished or abolished in such injected embryos (Fig. 2d). This was in contrast to embryos injected with sense MmGFP RNA or co-injected with both sense MmGFP mRNA and the "irrelevant" dsRNA for E-cadherin (Fig. 2a-b). Thus dsRNA can interfere both with the expression of a chromosomally located gene, and of synthetic mRNA introduced by microinjection.

### Example 2 - Phenocopying an E-cadherin knockout

We assessed the specific developmental consequences of injecting E-cadherin dsRNA. E-cadherin is both maternally and zygotically expressed during pre-implantation development. Disruption of the *E-cadherin* gene, using homologous recombination to remove regions of the molecule essential for adhesive function, leads to a severe pre-implantation defect. These embryos can initially undergo compaction, due to the presence of maternally expressed Ecadherin. However, they show a defect in cavitation and never form normal blastocysts (Larue, et al. Proc Natl Acad Sci U SA 91, 8263-8267 (1994); Riethmacher, et al. Proc Natl Acad Sci U S A 92, 855-859 (1995)).

We observed that following injection of *E-cadherin* dsRNA, the phenotype was identical to that of null mutant embryos. Thus, the embryos initially developed normally to the compaction stage of the morula (data not shown). However, only about 30% were able to cavitate, and formed the so called "cysts" but did not form normal blastocysts (Larue, et al Proc Natl Acad Sci U S A 91, 8263-8267 (1994)) (Table 1). In contrast, the great majority of uninjected embryos or control embryos injected with *MmGFP* dsRNA cavitated and formed normal blastocysts (Table 1).

**Table 1.**

| **Phenotypes obtained following injection of *E-cadherin* dsRNA into zygotes** | | | | |
|---|---|---|---|---|
| ***dsRNA injected*** | **No. of experiments** | **No. of embryos** | **Known null mutant phenotype** | **Phenotype resulting from *E cadherin* dsRNA injection** |
| None | 6 | 240 | > 90% formed blastocysts (Obsugi, et al. Dev Biol 185, 261-271 (1997)) | 91.6%±18.3% formed blastocysts |
| *gfp* (2 mg ml⁻¹) | 5 | 89 | N.A.* | 74.1%%±17% formed blastocysts |
| *Ecadherin* (2 mg ml⁻¹) | 5 | 130 | 47.5% formed cysts. Remaining failed to develop to this stage (Larue, et al Proc Natl Acad Sci USA 91, 8263-8267 (1994); Ohsugi, et al. Dev Biol 185, 261-271 (1997)) | 26.9%±25.6% formed cysts; Remaining failed to develop to this stage |

| | | | | |
|---|---|---|---|---|
| *N.A.: Not applicable. Mean± s.d. ^{a} Significantly different from results with *GFP* dsRNA using the χ2 test (p < 0.05). | | | | |

The analysis of E-cadherin expression by immunostaining and immunoblotting shows that the expression of E-cadherin is dramatically decreased after *E-cadherin* dsRNA injection (Fig. 3b, c). In contrast, no decrease in E-cadherin expression was observed in the embryos injected with *MmGFP* dsRNA, for which the level of E-cadherin expression was similar to that of the control uninjected embryos (Fig. 3c). The level of E-cadherin at the morula stage in embryos injected *with E-cadherin* dsRNA is lower than in newly fertilised embryos before injection (Fig. 3c). This residual E-cadherin protein may largely reflect persistence of maternally expressed protein whose synthesis ceases during the 2 cell stage (Sefton, et al, Development 115, 313-318 (1992)). This residual maternal protein is present until the late blastocyst stage in homozygous null embryos (Larue, et al Proc Natl Acad Sci U S A 91, 8263-8267 (1994)).

We conclude that injection of *E-cadherin* dsRNA leads to a striking reduction of E-cadherin protein and consequently a similar phenotype to that of the null mutant embryos.

### Example 3 - dsRNA interference in the oocyte

In orderto determine whether dsRNA might be used to interfere with maternally expressed genes, we sought a model gene having a characteristic knockout phenotype. C-mos is an essential component of cytostatic factor, responsible for arresting the maturing oocyte at metaphase in the second meiotic division. In *c-mos* -/- mice, between 60 and 75 % of oocytes do not maintain this metaphase II arrest and initiate parthenogenetic development (Colledge, et al, Nature 370, 65-68 (1994); Hashimoto, et al. Nature 370, 68-71 (1994)). *C-mos* mRNA is present in fully grown immature oocytes, and its translation is initiated from maternal templates when meiosis resumes following germinal vesicle breakdown (Verlhac, et al. Development 122, 815-822 (1996)). Thus, injection of *c-mos* dsRNA would allow us to test whether dsRNA could interfere with maternal mRNA expression.

When we injected *c-mos* dsRNA into oocytes, about 63 % did not maintain arrest in metaphase II (Table 2). Of these, 78% initiated parthenogenetic development and progressed to 2 to 4 cell stage embryos (Fig. 4a, b, c). The remainder underwent fragmentation. Both of these events occur at similar frequencies in null mutant oocytes (Colledge, et al, Nature 370, 65-68 (1994)). In contrast, only 1-2% of control oocytes, either uninjected or injected with *MmGFP* dsRNA, underwent spontaneous activation (Table 2). We were still able to observe that 42 % of injected oocytes failed to undergo metaphase II arrest, when we reduced the concentration of injected *c-mos* dsRNA by 20 fold to 0.1 mg/ml (Table 2). This is a significantly higher concentration than that believed to be effective in *C. elegans* and plants, where it is claimed that an effect can be achieved with a few molecules of dsRNA per cell.

**Table 2.**

| **Phenotypes observed following injections of *c-mos* dsRNA in the germinal vesicle stage oocyte** | | | | |
|---|---|---|---|---|
| ***DsRNA injected*** | **No. of experiments** | **No. of oocytes** | **Known null mutant phenotype** | **Phenotype resulting from *dsRNA* injections** |
| None | 1 | 158 | N.A* | 1.3%±2%spontaneous activation; 3.8%+5.8% fragmentation |
| *Dsgfp* (2 mg ml⁻¹) | 4 | 73 | N.A * | 1.4±2.1%spontaneous activation; 2.7±2% fragmentation |
| *Ds mos* (2 mg ml⁻¹) | 4 | 108 | 60-75% released from the metaphase II arrest. High degree of cytoplasmic fragmentation (Colledge, et al. Nature 370, 65-68 (1994); Hashimoto, et al. Nature 370, 68-71 (1994)) | 49.1 ± 27 %^{a} released from the metaphase II block; 13.9±13% fragmentation |
| *Ds mos* (0.1 mg ml⁻¹) | 2 | 33 | as above | 36.4±7.6%^{b} released from the metaphase II block; 6.1±1.9% fragmentation |

| | | | | |
|---|---|---|---|---|
| *N.A.: Not applicable. We observed that uninjected oocytes rarely underwent spontaneous activation and at a similar frequency to those injected with GFPdsRNA.mean ± s.d. ^{a}'^{b} Significantly different from results with *GFP dsRNA* using the χ2 test (p<0.05) | | | | |

We confirmed that c-mos dsRNA interferes with c-mos expression by immunoblot analysis carried out 12 hours after the injection of germinal vesicle stage oocytes before the phenotype consequences of its loss of expression become apparent (Fig. 4e). Thus, injection of *c-mos* dsRNA into the oocyte specifically interferes with *c-mos* activity to mimic the targeted deletion of *c-mos* via homologous recombination. These experiments show that dsRNA is able to block the expression of maternally provided gene products.

### Example 4 - the effects of RNAi are clonally inherited within the mouse embryo

To assess whether it would be possible to eliminate the expression of specific genes within defined lineages of cells within the early mouse embryo, dsRNA to *E-cadherin* was microinjected into one cell of a two cell stage mouse embryo, together with synthetic mRNA for MmGFP to mark the injected cell. The expression levels of E-cadherin and MmGFP was followed as these embryos developed. The expression of E-cadherin was reduced specifically in cells derived from the one injected with ds *E-cadherin* RNA, the clone being marked by the expression of MmGFP translated from the injected mRNA into the same cell. Thus, in the early mouse embryo, the effect of dsRNA is not transmitted to neighbouring cells. Thus, dsRNAi can be used in the embryo to regulate patterns of gene expression differentially between lineages having with different fates.

### Discussion

We have demonstrated that dsRNA can be used as a specific inhibitor of gene activity in the mouse oocyte and pre-implantation or early embryo. We show the specificity of the procedure by individually inhibiting the expression of 3 different genes: *c-mos* in the oocyte, and *E-cadherin* or a *gfp* transgene in the early embryo. In the cases of the two endogenous mouse genes, this results in phenotypes comparable to those of null mutants. Our experiments to prevent expression of the *gfp* transgene indicate that RNAi *per se* does not affect the normal course of development.

Two of our experiments support the hypothesis that RNAi acts in the mouse by either inducing degradation of the targeted RNA, or inhibiting its translation. First we show that injection of MmGFPdsRNA inhibits the expression of co-injected sense *MmGFP* mRNA. Secondly, we injected dsRNA against *c-mos* into oocytes before the germinal vesicle breaks down, the stage when *c-mos* mRNA has accumulated but has not yet been translated. C-mos is translated when the germinal vesicle breaks down, to arrest oocytes in metaphase II of the second meiotic division. We found that *c-mos* dsRNA prevents its function; oocytes proceed through metaphase II and undergo parthenogenetic activation. In each case, the effects of RNAi persist for sufficient time to phenocopy the loss of gene function. When dsRNA is introduced into early blastocysts, it remains effective until early post-implantation stages. The clonal inheritence of the RNAi effect indicates that it may be targeted towards a pattern of gene activity in a specific lineage. Finally, as RNAi functions in peri-implantation development, it may be expected to result in elimination of expression of target genes in embryonic stem cells established from mouse embryos at this developmental stage, and this may facilitate their directed differentiation into specific cell types.

## Claims

1. The use of an RNA in the manufacture of a medicament for inhibiting the expression of a target gene in a mammalian cell, wherein the target gene causes or is likely to cause disease and wherein the mammalian cell is an embryonic stem cell of a non-human pre-implantation embryo, the RNA comprising a double stranded structure having a nucleotide sequence, which nucleotide sequence is 100% identical to at least a part of said target gene in said mammalian cell which double stranded structure is at least 25 bases long, and which nucleotide sequence is derived from an endogenous template, and wherein the RNA comprises two separate complementary RNA strands.

2. The use as claimed in claim 1, wherein the target gene is an endogenous gene.

3. The use as claimed in claim 1, wherein the target gene is a viral gene.

4. The use as claimed in claim 1, wherein the pre-implantation embryo is a blastocyte.

## Patentansprüche

1. Verwendung einer RNA in der Herstellung eines Medikaments zum Inhibieren der Expression eines Zielgens in einer Säugerzelle, wobei das Zielgen Krankheit verursacht oder es wahrscheinlich ist, dass es Krankheit verursacht, wobei die Säugerzelle eine embryonale Stammzelle eines nicht humanen Präimplantationsembryos ist, wobei die RNA eine doppelsträngige Struktur umfasst, die eine Nukleotidsequenz hat, die zu 100% mit zumindest einem Teil des Zielgens in der Säugerzelle identisch ist, wobei die doppelsträngige Struktur mindestens 25 Basen lang ist, die Nukleotidsequenz von einem endogenen Muster abgeleitet ist, und die RNA zwei getrennte komplementäre RNA-Stränge umfasst.

2. Verwendung nach Anspruch 1, wobei das Zielgen ein endogenes Gen ist.

3. Verwendung nach Anspruch 1, wobei das Zielgen ein virales Gen ist.

4. Verwendung nach Anspruch 1, wobei der Präimplantationsembryo eine Blastocyste ist.

## Revendications

1. Utilisation d'un ARN dans la fabrication d'un médicament destiné à inhiber l'expression d'un gène cible dans une cellule de mammifère, dans laquelle le gène cible provoque ou est susceptible de provoquer une maladie et dans laquelle là cellule de mammifère est une cellule souche embryonnaire d'un embryon préimplantatoire non humain, l'ARN comprenant une structure à double brin ayant une séquence de nucléotides, ladite séquence de nucléotides présente une identité de 100 % à une partie dudit gène cible dans ladite cellule de mammifère, ladite structure à double brin est longue d'au moins 25 bases, et ladite séquence de nucléotides est dérivée d'une matrice endogène, et dans laquelle l'ARN comprend deux brins ARN complémentaires séparés.

2. Utilisation selon la revendication 1, dans laquelle le gène cible est un gène endogène.

3. Utilisation selon la revendication 1, dans laquelle le gène cible est un gène viral.

4. Utilisation selon la revendication 1, dans laquelle l'embryon préimplantatoire est un blastocyte.
